# EUROPÄISCHE PATENTANMELDUNG

(11) **EP 2 899 533 A1**
(43) Veröffentlichungstag der Anmeldung: **29.07.2015**
(21) Anmeldenummer: 14152041.1
(22) Anmeldetag: 22.01.2014
(51) Int. Cl.: G01N 21/39, G01J 3/433

(54) **Verfahren zur Wellenlängenmodulationsspektroskopie mit einem Filter für das demodulierte Messsignal und das simulierte Signal**

(71) Anmelder: Siemens Aktiengesellschaft, 80333 München (DE)
(72) Erfinder: Steinbacher, Franz, 76137 Karlsruhe (DE); Bitter, Ralf, 76139 Karlsruhe (DE); Hankiewicz, Thomas, 76149 Karlsruhe (DE); Marquardt, Christoph Wolfgang, 76149 Karlsruhe (DE); Nygren, Jan, 76137 Karlsruhe (DE); Pleban, Kai-Uwe, 76297 Stutensee (DE); Mucha, Adrian, 76187 Karlsruhe (DE)

(57) **Zusammenfassung**

Bei einem auf der Wellenlängen-Modulationsspektroskopie (WMS) beruhenden Verfahren zur Gasanalyse wird das erhaltene Messsignal (13) bei einer Oberschwingung der Modulationsfrequenz demoduliert und durch Anfitten einer Sollkurve (19) an den Verlauf des demodulierten Messsignals (13') zu einem Messergebnis (17) ausgewertet.

Um aus Störeinflüssen (z. B: Temperaturänderungen, Änderung der Konzentration von Störgasen) resultierende Änderungen in den Messergebnissen zu reduzieren, werden sowohl das demodulierte Messsignal (13') als auch die Sollkurve (19) mit derselben Filterfunktion (Filter 15, 20) gefiltert, wobei die Filterfunktion zur Unterdrückung von Störsignalanteilen ausgelegt ist, die sowohl mit Nutzsignalanteilen des demodulierten Messsignals (13') als auch mit der Sollkurve (19) interferieren.

## Beschreibung

Die Erfindung betrifft ein Verfahren zur Messung der Konzentration einer Gaskomponente in einem Messgas mittels eines Gasanalysators, wobei
zur wellenlängenabhängigen Abtastung einer interessierenden Absorptionslinie der Gaskomponente die Wellenlänge des Lichts einer wellenlängenabstimmbaren Lichtquelle innerhalb periodisch aufeinanderfolgender Abtastintervalle variiert und dabei zusätzlich mit einer Frequenz moduliert wird,
das modulierte Licht durch das Messgas auf einen Detektor geführt wird,
ein von dem Detektor erzeugtes Messsignal bei einer Oberschwingung der Frequenz demoduliert wird, und durch Anfitten einer Sollkurve an den Verlauf des demodulierten Messsignals ein Messergebnis erzeugt wird.

Ein derartiges Verfahren ist aus der EP 1 475 618 B1 bekannt.

Bei dem bekannten Verfahren erzeugt eine wellenlängenabstimmbare Lichtquelle in Form einer Laserdiode Licht im Infrarotbereich, das durch ein zu messendes Prozessgas (Messgas) geleitet und anschließend detektiert wird. Die Wellenlänge des Lichts wird auf eine spezifische Absorptionslinie der jeweils zu messenden Gaskomponente abgestimmt, wobei die Absorptionslinie periodisch wellenlängenabhängig abgetastet wird. Dazu wird die Laserdiode innerhalb von periodisch aufeinanderfolgenden Abtastintervallen mit einem rampen- oder dreieckförmigen Stromsignal angesteuert. Während der vergleichsweise langsamen Abtastung der Absorptionslinie wird zusätzlich die Wellenlänge des erzeugten Lichts mit hoher Frequenz und kleiner Amplitude sinusförmig moduliert. Da das Profil der Absorptionslinie nicht linear ist, werden in dem bei der Detektion erhaltenen Messsignal auch Harmonische oberhalb der Modulationsfrequenz erzeugt. Das Messsignal wird üblicherweise bei einer n-ten Oberschwingung, vorzugsweise der zweiten Harmonischen, durch phasensensitive Lock-in Technik demoduliert und für jedes Abtastintervall zu einem Messergebnis ausgewertet. Bei kleiner Modulationsamplitude ist die Detektion der n-ten Harmonischen direkt proportional zu der n-ten Ableitung des direkten Messsignals. Die Auswertung erfolgt z. B. durch Anfitten (Curve-Fitting) des im Idealfall zu erwartenden Verlaufs des demodulierten Messsignals (Sollkurve) an dessen tatsächlichen Verlauf (Istkurve). Aus dem dabei erhaltenen Messergebnis wird schließlich die Konzentration der zu messenden Gaskomponente bestimmt.

Temperaturänderungen innerhalb des Gasanalysators können zu Änderungen der Messergebnisse führen. Diese als Drift bezeichnete Charakteristik des Gasanalysators schränkt sein Messverhalten und zu realisierende Applikationen maßgeblich ein. Eine Ursache für die Drift können unter anderem Etalons im optischen Strahlengang sein. Diese führen in dem Verlauf des demodulierten Messsignals zu periodischen Strukturen, die im Frequenzbereich des zu erwartenden Absorptionssignals liegen. Beim Curve-Fitting führt dies zu fehlangefitteten Funktionen und Abweichungen zwischen der ermittelten Konzentrationen von der tatsächlichen Konzentrationen der zu messenden Gaskomponente.

Zur Unterdrückung dieser Störsignalanteile ist es aus der oben genannten EP 1 475 618 B1 bekannt, einen Teil des von der Lichtquelle erzeugten Lichts unmittelbar auf einen Monitordetektor zu führen und das erhaltene Monitorsignal bei der n-ten Oberschwingung zu demoduliert und auszuwerten. Jede Abweichung des demodulierten Monitorsignals von einer Nulllinie beruht auf einer optischen Störung, die, soweit sie im Bereich der Lichtquelle oder in dem von Mess- und Monitorkanal gemeinsamen genutzten Wegabschnitt des Strahlengangs liegt, auch das Messsignal beeinträchtigt. Diese Störung wird durch eine Vorverzerrung der Ansteuerung der Lichtquelle kompensiert, indem die Wellenlänge des Lichts zusätzlich mit der n-ten Oberschwingung moduliert wird, wobei die Modulationsintensität von dem demodulierten Monitorsignal abhängig ist.

Die Auskopplung eines Teils des erzeugten Lichts auf den Monitordetektor ist jedoch mit einem erhöhten konstruktiven und schaltungstechnischen Aufwand verbunden, der mit einer höheren Störempfindlichkeit einhergeht. Außerdem können außerhalb der gemeinsamen Abschnitte des Mess- und Monitorkanals auftretende Störungen des Messsignals nicht kompensiert werden.

Aus der EP 2 336 738 A1 oder EP 1 927 831 A1 ist es bekannt, die optische Weglänge beispielsweise durch mechanische Vibration der Lichtquelle zu variieren und die störenden periodischen Strukturen aus dem demodulierten Messsignal herauszumitteln. Dadurch lassen sich aber nur bestimmte, von parallelen optischen Oberflächen im Strahlengang erzeugte Interferenz-Störungen reduzieren.

Der Erfindung liegt die Aufgabe zugrunde, aus Störeinflüssen wie z. B. Temperaturänderungen in dem Gasanalysator resultierende Änderungen in den Messergebnissen zu reduzieren.

Gemäß der Erfindung wird die Aufgabe dadurch gelöst, dass bei dem Verfahren der eingangs angegebenen Art sowohl das demodulierte Messsignal als auch die Sollkurve mit jeweils derselben Filterfunktion gefiltert werden, wobei die Filterfunktion dazu ausgelegt ist, mit Nutzsignalanteilen des demodulierten Messsignals und mit der Sollkurve interferierende Störsignalanteile des demodulierten Messsignals zu unterdrücken.

Treten die Störungen, wie die oben erwähnten Etalon-Störungen, hauptsächlich bei bestimmten Frequenzen auf, so ist es möglich, diese durch Filterung zu dämpfen oder aus dem demodulierten Messsignal zu entfernen. Überlappen sich die Spektren der Stör- und Nutzsignalanteile des Messsignals, beeinflusst das Filter auch den Nutzsignal; d. h. das gefilterte Nutzsignal ändert durch die Filterung seine Form. Die Sollkurve, auf die gefittet werden soll, wird daher gemäß der Erfindung derselben Filterfunktion gefiltert wie das demodulierte Messsignal. Die gefilterte Sollkurve wird auf das gefilterte demodulierte Messsignal gefittet. Zweck der Filterung ist es, die Störsignalanteile stärker zu dämpfen als die Nutzsignalanteile und so eine Verbesserung des Nutz-/ Störsignal-Abstands zu erreichen. Dies ist grundsätzlich dann möglich, wenn die Spektren der Stör- und Nutzsignalanteile nicht gleich sind. So ist der Störsignalanteil eines Etalons breiter ist als der Nutzsignalanteil und enthält dementsprechend mehr niederfrequente Anteile als das Nutzsignal.

Entsprechend der Erfindung ist die Filterfunktion dazu ausgelegt, die mit den Nutzsignalanteilen des demodulierten Messsignals und mit der Sollkurve interferierenden Störsignalanteile des demodulierten Messsignals zu unterdrücken. Soweit das demodulierte Messsignal bisher schon gefiltert wurde, erfolgte die Filterung dagegen recht breitbandig, weil sich das Spektrum mit der Breite der Absorptionslinie ändert, die wiederum vom Druck abhängig ist. Die Bandbreite der bisherigen Filterung wurde daher so gewählt, dass sowohl schmale als auch breite demodulierte Messsignale nicht gestört wurden. Der Curve-Fit-Algorithmus wird jedoch von Frequenzen, die nicht in dem zu fittenden Signal enthalten sind, kaum gestört und hat somit selbst eine gute Filterwirkung. Das bedeutet, dass eine genaue Anpassung des Filters bzw. der Filterfunktion an die Sollfunktion (Matched Filter) im Unterschied zu der erfindungsgemäßen Filterung nur zu geringen Verbesserungen führen würde, wobei die störenden Frequenzen weiterhin wirksam wären, weil sie in demselben Bereich wie das Nutzsignal liegen.

Da die Störungen hauptsächlich im niederfrequenten Bereich auftreten, wird vorzugsweise die Filterfunktion eines Hochpassfilters verwendet. Der Nachteil dieses Verfahrens ist ein niedrigerer Signal-Rauschabstand bei konstanter Umgebungstemperatur, da Signalanteile des Nutzsignals weggefiltert werden. Dadurch wird die Temperaturabhängigkeit auf Kosten der Nachweisgrenze bei konstanter Umgebungstemperatur reduziert. Da jedoch die Temperaturabhängigkeit deutlich größer ist als das Rauschen bei konstanter Umgebungstemperatur, kann dessen Nachteil vernachlässigt werden. Davon abgesehen kann Rauschen durch Mittelung oder Kalman-Filterung relativ einfach unterdrückt werden, wohingegen Etalon-Störungen nur schwer nachträglich entfernt werden können.

Zur weiteren Erläuterung der Erfindung wird im Folgenden auf die Figuren der Zeichnung Bezug genommen; im Einzelnen zeigen:
- Figur 1: ein Ausführungsbeispiel eines Gasanalysator zur Durchführung des erfindungsgemäßen Verfahrens,
- Figur 2: ein Beispiel für ein gestörtes demoduliertes Messsignal vor und nach seiner Filterung und ,
- Figur 3: ein Beispiel für das Curve-Fitting bei ungefiltertem demoduliertem Messsignal, und
- Figur 4: ein Beispiel für das Curve-Fitting bei dem gefilterten demodulierten Messsignal.

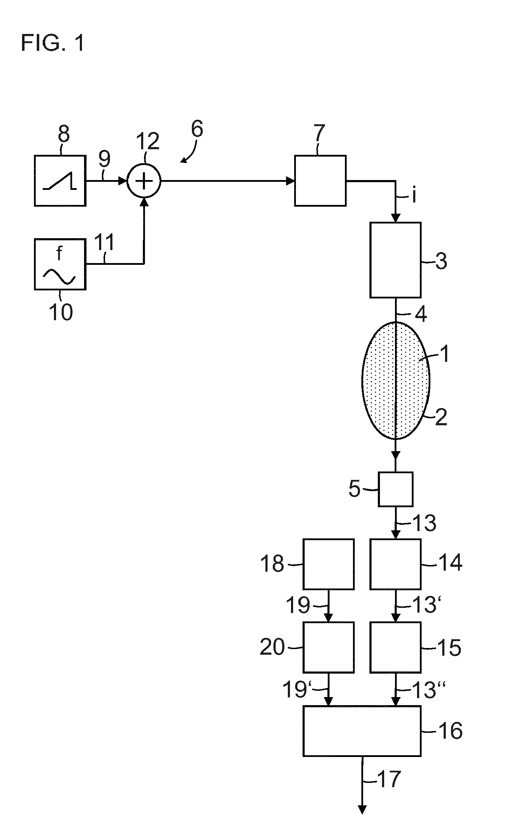

Bei dem in Figur 1 in Form eines vereinfachten Blockschaltbildes gezeigten Gasanalysator handelt es sich um ein Laserspektrometer zur Messung der Konzentration mindestens einer interessierenden Gaskomponente eines Messgases 1, das in einem Messvolumen 2, beispielsweise einer Messküvette oder einer Prozessgasleitung, enthalten ist. Das Spektrometer enthält eine Lichtquelle 3 in Form einer Laserdiode, deren Licht 4 nach Durchstrahlen des Messgases 1, auf einen Messdetektor 5 fällt. Eine von einer Modulationseinrichtung 6 gesteuerte Stromquelle 7 speist die Laserdiode 3 mit einem Injektionsstrom i, wobei die Intensität und Wellenlänge des erzeugten Lichts 4 von dem Strom i und der Betriebstemperatur der Laserdiode 3 abhängen. Die Modulationseinrichtung 6 umfasst einen ersten Signalgenerator 8, der die Stromquelle 7 periodisch mit einer vorgegebenen, vorzugsweise rampen- oder dreieckförmigen Funktion 9 angesteuert, um mit der mehr oder weniger linear dem Verlauf des Stromes i folgenden Wellenlänge des erzeugten Lichts 4 eine ausgewählte Absorptionslinie der interessierenden Gaskomponente abzutasten. Ein zweiter Signalgenerator 10 erzeugt ein sinusförmiges Signal 11 höherer Frequenz f₀, mit dem in einem Summierglied 12 die rampen- oder dreieckförmige Funktion 9 moduliert wird.

Der Messdetektor 5 erzeugt in Abhängigkeit von der detektierten Lichtintensität ein Messsignal 13, das in einem Lock-in-Verstärker 14 bei einer Harmonischen nf₀ (n = 1, 2, 3...), hier z. B. 2f₀, der Modulationsfrequenz f₀ demoduliert wird. Das demodulierte Messsignal 13' wird in einem Filter 15 mit einer vorgegebenen, festen oder z. B. temperaturabhängig variierbaren Filterfunktion gefiltert, um Störsignalanteile zu unterdrücken. In einer nachgeordneten Auswerteeinrichtung 16 wird das gefilterte demodulierte Messsignal 13" für jedes Abtastintervall zu einem Messergebnis 17 ausgewertet. Dazu wird durch eine Einheit 18 eine dem idealen demodulierten Messsignal 13' entsprechende Sollkurve 19 bereitgestellt, die in einem Filter 20 mit derselben Filterfunktion wie die des Filters 15 gefiltert wird; die dabei erhaltene gefilterte Sollkurve 19' wird anschließend in der Auswerteeinrichtung 16 an das gefilterte demodulierte Messsignal 13" angefittet.

Wie eingangs bereits erläutert, können Temperaturänderungen innerhalb des Gasanalysators zu einer Drift der Messergebnisse 17 führen, wobei eine Ursache für die Drift Etalons im optischen Strahlengang sind, die zu periodischen Strukturen im Verlauf des demodulierten Messsignals 13" führen. Ferner kann das demodulierten Messsignals 13" verrauscht sein.

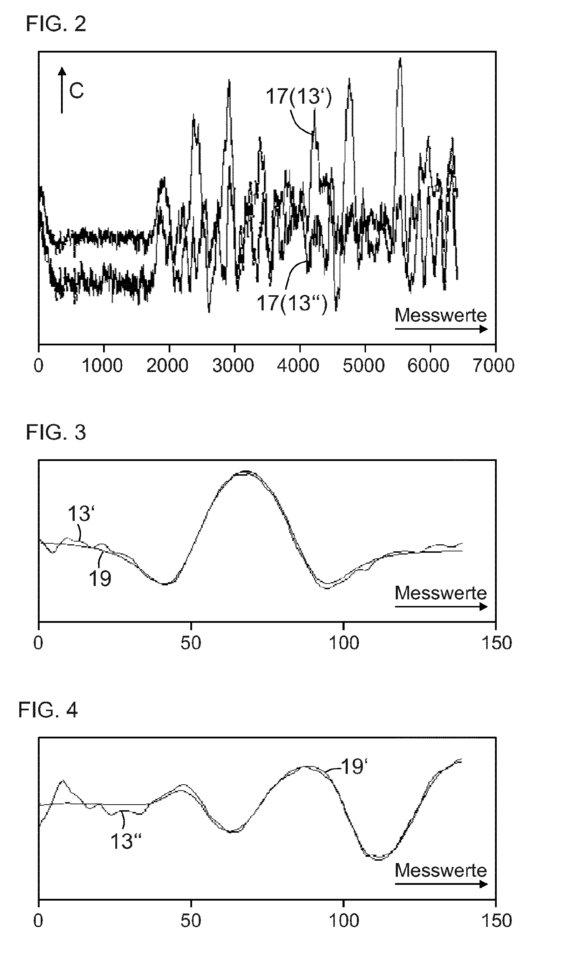

Figur 2 zeigt beispielhaft die über eine Messreihe von etwa 7000 Abtastintervallen erhaltenen Messergebnisse, hier der Konzentrationswert C der zu messenden Gaskomponente, wobei der obere Kurvenverlauf 17(13') die Messergebnisse zeigt, die ohne die erfindungsgemäße Filterung des demodulierten Messsignals 13' erhalten werden, und der untere Kurvenverlauf 17(13") die Messergebnisse zeigt, die aufgrund des gemäß der Erfindung gefilterten, hier z. B. hochpassgefilterten, demodulierten Messsignals 13" erhalten werden. Man erkennt einen ersten, etwa 1700 Messwerte umfassenden Bereich, in dem die Temperatur konstant gehalten wurde und der gemessene Konzentrationswert C im Wesentlichen durch Rauschen beeinflusst ist. In dem sich anschließenden Bereich wurde die Temperatur rampenförmig erhöht, wobei sich die Etalon-Störungen stark bemerkbar machen. Da durch die erfindungsgemäße Filterung auch Signalanteile des Nutzsignals weggefiltert werden, ist das Rauschen der gefilterten Messergebnisse 17(13") etwas größer als das der ungefilterten Messergebnisse 17(13'). Dadurch wird die Temperaturabhängigkeit auf Kosten der Nachweisgrenze bei konstanter Umgebungstemperatur reduziert. Der Effekt ist jedoch von vernachlässigbarer Größe, zumal das Rauschen durch Mittelung oder Kalman-Filterung relativ einfach unterdrückt werden. Andererseits sind aber die Etalon-Störungen bei den gefilterten Messergebnissen 17(13")) erheblich geringer als das bei den ungefilterten Messergebnissen 17(13').

Figur 3 zeigt ein Beispiel für das herkömmliche Anfitten der dem idealen demodulierten Messsignal 13' entsprechenden Sollkurve 19 an das reale demodulierte Messsignal 13'.

Figur 4 zeigt ein Beispiel für das erfindungsgemäße Anfitten der gefilterten Sollkurve 19' an das reale gefilterte demodulierte Messsignal 13".

## Patentansprüche

1. Verfahren zur Messung der Konzentration einer Gaskomponente in einem Messgas (1) mittels eines Gasanalysators, wobei zur wellenlängenabhängigen Abtastung einer interessierenden Absorptionslinie der Gaskomponente die Wellenlänge des Lichts (4) einer wellenlängenabstimmbaren Lichtquelle (3) innerhalb periodisch aufeinanderfolgender Abtastintervalle variiert und dabei zusätzlich mit einer Frequenz (f₀) moduliert wird,
das modulierte Licht (4) durch das Messgas (1) auf einen Detektor (5) geführt wird,
ein von dem Detektor (5) erzeugtes Messsignal (16) bei einer Oberschwingung (nf₀) der Frequenz (f₀) demoduliert wird, und durch Anfitten einer Sollkurve (19) an den Verlauf des demodulierten Messsignals (13') ein Messergebnis erzeugt wird, **dadurch gekennzeichnet,**
**dass** sowohl das demodulierte Messsignal (13') als auch die Sollkurve (19) mit derselben Filterfunktion gefiltert werden, wobei die Filterfunktion dazu ausgelegt ist, mit Nutzsignalanteilen des demodulierten Messsignals (13') und mit der Sollkurve (19) interferierende Störsignalanteile des demodulierten Messsignals (13') zu unterdrücken.

2. Verfahren nach Anspruch 1, **dadurch gekennzeichnet, dass** die Filterfunktion eines Hochpassfilters verwendet wird.
